# EUROPEAN PATENT APPLICATION

(11) **EP 2 033 581 A1**
(43) Date of publication of application: **11.03.2009**
(21) Application number: 07115951.1
(22) Date of filing: 07.09.2007
(51) Int. Cl.: A61B 17/00

(54) **Prosthetic valve delivery system including retrograde/antegrade approch**

(71) Applicant: SORIN BIOMEDICA CARDIO S.R.L., 13040 Saluggia (Vercelli) (IT)
(72) Inventor: Suri, Rakesh Mark, Rochester, MN 55902 (US); Vallana, Franco, 10123, Torino (IT); Rolando, Giovanni, 10034, Chivasso (Torino) (IT); Righini, Giovanni, 10034, Chivasso (Torino) (IT); Phillips, Brent Russell, Rochester, MN 55906 (US)
(74) Representative: Bosotti, Luciano

(57) **Abstract**

A device for implanting an expandable heart valve prosthesis at a valve annulus in a patient's heart includes an implantation device configured to extend from a first opening in the patient's body, through the patient's aorta, through a valve annulus, through an opening in a ventricle, and to exit through the patient's thoracic region. The device further includes a port having a hemostasis valve for providing access through the patient's thoracic region and into the ventricle and at least two interchangeable modules configured for delivery using the implantation device.

## Description

### TECHNICAL FIELD

The present invention relates generally to methods and systems for cardiovascular surgery. More particularly, the invention relates to methods and systems for the repair, removal and/or replacement of heart valves.

### BACKGROUND

Minimally-invasive percutaneous valve replacement procedures have emerged as an alternative to open-chest surgery, using intravascular catheterization (e.g., from a femoral artery access point) or a minimally-invasive surgical technique. Because the minimally-invasive approach requires only a small incision, it allows for a faster recovery for the patient with less pain and bodily trauma. This, in turn, reduces the medical costs and the overall disruption to the life of the patient.

An inherent difficulty in the minimally-invasive percutaneous approach is the limited space that is available within the vasculature. Unlike open heart surgery, minimally-invasive heart surgery offers a surgical field that is only as large as the diameter of a blood vessel. Consequently, the introduction of tools and prosthetic devices is challenging. The device must be dimensioned and configured to permit it to be introduced into the vasculature, maneuvered therethrough, and positioned at a desired location. Moreover, lesions located in the vasculature may be a contraindication for certain types of delivery systems using femoral access.

Thus, there is a need in the art for methods and devices for performing heart valve repair and replacement, as well as other procedures within the heart and great vessels, that provide greater ease of access to native heart valves.

### BRIEF DESCRIPTION OF THE DRAWINGS

FIG. 1 is a cross-sectional view of a human heart including a prosthetic heart valve.

FIG. 2 is a schematic view of the left side of the heart including an implantation device according to an embodiment of the present invention.

FIG. 3 is a schematic view of the left side of the heart including an implantation device and one interchangeable module according to an embodiment of the present invention.

FIGS. 4A and 4B are schematic views of the of the left side of the heart including an implantation device and another interchangeable module according to another embodiment of the present invention.

FIG. 5 is a schematic view of the left side of the heart including an implantation device and two interchangeable modules according to yet another embodiment of the present invention.

FIG. 6 is a schematic view of the left side of the heart including an implantation device and yet another interchangeable module according to yet another embodiment of the present invention.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

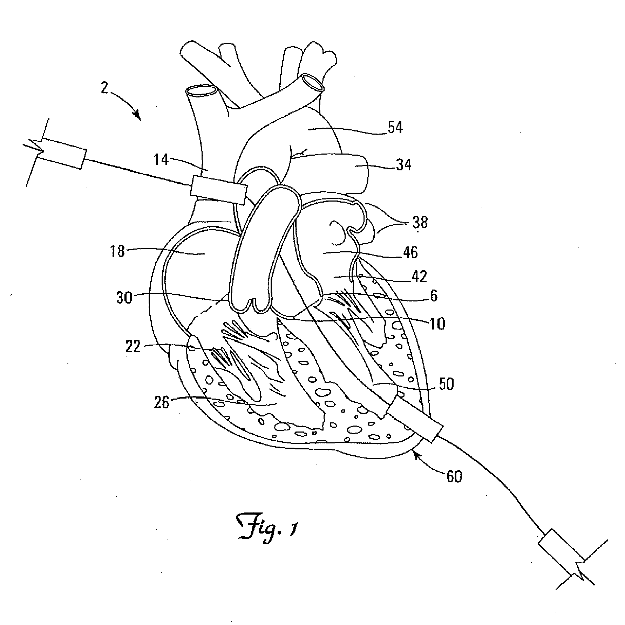

FIG. 1 is a cross-sectional view of a human heart 2 with an expandable prosthetic heart valve 6 implanted within or adjacent an aortic valve annulus 10. Blood flows from the superior and inferior vena cavas 14 into a right atrium 18 of the heart 2 and then flows through a tricuspid valve 22 into a right ventricle 26. A pulmonary valve 30 facilitates blood flow from the right ventricle 26 to the pulmonary arteries 34. The blood is then oxygenated by the lungs and returned back to the heart via pulmonary veins 38. A mitral valve 42 then facilitates blood flow from a left atrium 46 to a left ventricle 50. Blood then flows from the left ventricle 50, through the aortic valve annulus 10, to the aorta 54. The aorta 54 then delivers the blood to the coronary arteries and the peripheral vascular system.

Using techniques well known to those of skill in the art, access to the heart 2, according to some embodiments, is established percutaneously through the aorta, femoral, radial or brachial arteries using a retrograde approach to reach a target site within the heart 6 (e.g. the aorta 54 or aortic valve annulus 10). Likewise, according to some embodiments, access to the aortic valve annulus is accomplished using a retrograde, minimally-invasive surgical technique to provide access to the aortic valve annulus 10 through an opening in the aortic arch.

Alternatively, according to various embodiments, access to the heart 2 and the aortic valve annulus 10 is established through the apical area 60 (e.g., apex) of the heart. In these embodiments, a percutaneous access port may be established through a patient's chest wall and into the left ventricle at or near the apex 60. According to some embodiments, access to the left ventricle 50 through the apex 60 is established using one of the methods and devices generally shown and described in U.S. Patent 6,010,531, entitled "Less-invasive Devices and Methods for Cardiac Valve Surgery", which is incorporated herein by reference.

The apex 60 of the heart 2 is generally the blunt rounded inferior extremity of the heart 2 formed by the left and right ventricles, 26 and 50. In normal healthy humans, the apex 60 generally lies behind the fifth left intercostal space from the mid-sternal line. Access to the valve annulus 10 through the apex 60 can be established using a larger diameter access port than is possible using percutaneous access methods. Thus, apical access to the heart 2 permits greater flexibility with respect to the types of devices and surgical methods that may be performed in the heart and great vessels.

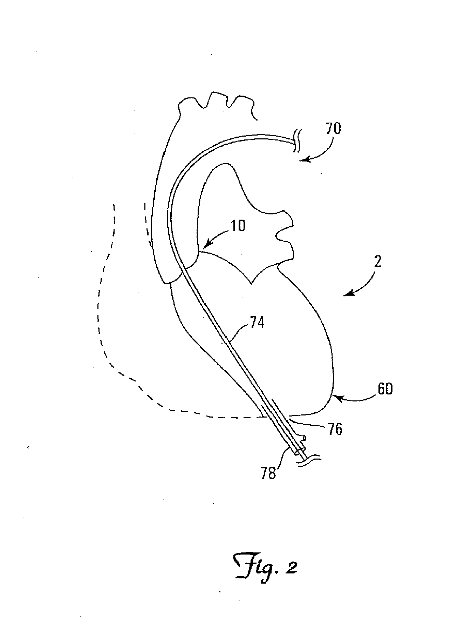

FIGS. 2-6 show schematic views of exemplary embodiments of a valve access system 70 for delivering a replacement heart valve to a target site in a patient's heart 2. The valve access system 70 of the present invention allows for an antegrade as well as a retrograde approach to a target site within the heart 2. Exemplary target sites include native heart valves (e.g. pulmonary, mitral, aortic, and tricuspid) needing repair and/or replacement. While FIGS. 2-6 show the valve access system 70 traveling through the aortic arch, in alternative embodiments, the system 70 could exit the vasculature through the aorta and patient's body through a slit or opening in the wall of the aortic vessel.

According to the embodiment of FIG. 2, the valve access system 70 includes an implantation device 74 and is configured to deliver a prosthetic heart valve to a target site within a patient's heart 2. The implantation device 74 is configured to serve as a rail or track for providing access to the target site from a location outside the patient's body. According to one embodiment of the present invention, the implantation device 74 is a thin, flexible guidewire or a somewhat thicker wire, such as a stylet. According to an alternative embodiment of the present invention, the implantation device is a guide catheter. The guide catheter is adapted to provide passage to a variety of devices compatible with the valve access system of the present invention. According to one embodiment, the implantation device is reversibly lockable.

The implantation device 74 is of sufficient size and length to pass through a first opening in a patient's body (e.g., an aorta or a femoral artery access point), through a patient's aorta 54, and to exit through an access port 76 established in a patient's left ventricle 50 at or near the apex 60. The implantation device 74 then extends through a transthoracic port 78, which provides access through the patient's thoracoabdominal (e.g., intercostal) region and into the left ventricle 50 at or near the apex 60. The transthoracic port according to various exemplary embodiments is one of an introducer, trocar, or cannula, as is generally known in the art. According to one exemplary embodiment of the present invention, the port 78 includes one or more hemostasis valves or seals. The hemostasis valve or seal is adapted to provide a blood tight seal against any blood loss or leakage during the procedure, and can be used at the apex, at the aorta, or in both locations. The port 78 is configured to allow passage of the implantation device, catheter, or any tools or devices to be delivered to the target site using the implantation device, while at the same time providing a blood tight seal against blood loss or leakage.

According to one exemplary technique for introducing the access system 70, a physician establishes an access port into the patient's aorta or femoral artery using any of a variety of well-known techniques. The physician then establishes a second access point in the patient's intercostal region using the port 78. Using known imaging and guidance techniques, the physician then advances the implantation device 74 through the patient's aorta or arterial system to the aortic valve annulus 10. The physician then advances the implantation device 74 across the aortic valve annulus 10, through the left ventricle 50, and out of the heart through the port 78. At this point, the physician has established a continuous pathway allowing both retrograde (i.e., through the aorta or aortic arch) and antegrade (i.e., through the apex 60) access to the aortic valve annulus 10.

According to another exemplary embodiment, the implantation device 74 includes two portions. The physician introduces a first portion through the aorta or femoral artery to the aortic valve annulus 10 and introduces a second portion through the apex 60. According to various embodiments, the physician then couples the two portions together at or near the valve annulus 10 or the aortic root. In these embodiments, the implantation device 74 includes any of a variety of known structures for coupling the free ends to one another, so as to form a continuous rail or track. According to other embodiments, the physician employs any of a numerous variety of techniques known in the art to introduce the implantation device 74. According to various embodiments, the physician employs one or more of the following techniques to implant the device 74: optionally ballooning the native valve in one variant of the invention, or removing the native valve (e.g., by cutting the valve out of the annulus), optionally using an umbrella capture feature to catch any debris that may result from the cut, and implanting the device 74.

Once introduced, the implantation device 74 establishes a pathway between a first opening and second opening in a patient's body. The implantation device is left in place within the heart 2 to provide a tether or "rail" over which other tools or devices may be delivered. Additional tools and devices may be delivered along the rail using either a retrograde (i.e., via the aorta) or an antegrade (i.e., via the apex and left ventricle) approach. The implantation device 74 allows for one or more than one device to be delivered to the target site at the same time or substantially at the same time. This feature allows flexibility in delivery options and may increase the speed in the successive steps in the valve replacement procedure.

By way of further example, one tool or set of tools can be delivered through the access port from the aorta while a second tool or set of tools can be delivered through the access point in the left ventricle, along a single track spanning both points of access or along separate tracks, one for each access point. An aortic valve (which can be stenotic in many patients) cutter or remover is delivered through the access point in the aorta by a first physician or technician. The native stenotic valve (e.g., aortic valve) is excised, stored in a chamber of the cutter, and then the cutter assembly is removed through the aortic access point. A delivery system for an expandable valve prosthesis can be guided through the ventricular access port and be positioned in the ventricle ready for immediate insertion into position once the cutting operation has been completed. The delivery system can be optionally operated by a second physician or technician and be ready and waiting for deployment. It is appreciated that the speed with which both the cutting and the valve prosthesis deployment operations is greatly increased and the procedure time is greatly reduced using a combination antegrade and retrograde delivery approach. In this way the hemodynamic blood flow for the patient is maintained at an acceptable level while the patient is off-pump or partially off-pump. Similarly, it is appreciated that a variety of tools or modules (or portions thereof) can be guided either antegrade or retrograde along the guidewire.

In various exemplary embodiments of the invention, the cutter or native valve leaflet remover is sized and dimensioned to be delivered through the apex of the heart, while the delivery system including a heart valve prosthesis is sized and dimensioned for delivery through an access point on the aorta. In yet another variant of the invention, an access point can be established at any suitable point in the aortal tree.

By way of further example, a top portion of the cutter can be guided into cutting position along the guidewire along from the aortic access point, while a bottom portion of a cutter can be guided along the guidewire through the access point in the ventricle. The two portions meet at the native valve and perform the cutting and removal operation and then are each removed through the same access areas where they entered the patient.

By way of further example, other modules that can travel along the guidewire can include pumps for assisting in maintaining the patient's hemodynamic flow. The pumps can be positioned in various locations along the guidewire as needed. Other modules (e.g., a cutter or prosthesis delivery module) can then be guided over the pumps to perform their required operations.

As described, the valve access system 70 allows for delivery of a variety of devices to or near the aortic valve annulus 10. The system 70 may be used for example to introduce any of a variety of prosthetic heart valves, including, for example, stented and stentless tissue valves. The system 70 may also be used to introduce an imaging system to view all or a portion of a procedure. Imaging systems are well-known in the art and include, for example, transesophageal echo, transthoracic echo, intravascular ultrasound imaging (IVUS), and/or a radiopaque dye or contrast fluid. The imaging system may also include an optical viewing device, such as a fiber-optic camera.

The valve access system 70 allows for delivery of a variety of additional tools or devices, including, for example, the following: leaflet excision tools, leaflet capture devices, imaging devices, prosthetic heart valves, prosthetic valve delivery systems, blood-pump devices, inflation catheters and balloons, debris capture collapsible and expandable umbrellas, stenotic tissue debridement tools, markers located on the guidewire to assist in location of the guidewire at the appropriate location, centering balloons to center the guidewire in the desired orientation, and the like. In yet a further variant, an anti-embolization module is also added. According to various embodiments, the leaflet excision module includes a rigid portion having an actuator exerting sufficient force to cut through a calcified leaflet.

According to another embodiment of the present invention, the valve access system includes one or more interchangeable modules that are configured to be delivered along the implantation device 74 to the valve annulus 10. The implantation device 74 permits one or more modules to be in use at a given time, in series or in parallel. Depending upon the size of the module, an antegrade, retrograde, or combination antegrade and retrograde delivery approach may be chosen. As the apex 60 is not limited by the size constraints which are presented by percutaneous surgical methods, larger modules or devices may be delivered through the port 78. Smaller modules may continue to be delivered through the femoral artery approach or directly through a port or incision in the aorta. Ultimately either approach may be chosen at the discretion of one of skill in the art performing the procedure.

In yet another embodiment of the invention, all modules are de-aired, filled with a fluid (e.g., saline), or filled with an appropriate gas (e.g., carbon dioxide or nitrogen) prior to use. This process eliminates the risk of an embolism occurring through the introduction of any of the modules described below directly into the beating heart of the subject. According to various embodiments, the modules are de-aired using one of the techniques described in co-pending, commonly assigned U.S. patent application number XX/YYY,YYY, filed on even date herewith, entitled "Fluid-Filled Delivery System for in Situ Deployment of Cardiac Valve Prostheses," which is hereby incorporated by reference in its entirety.

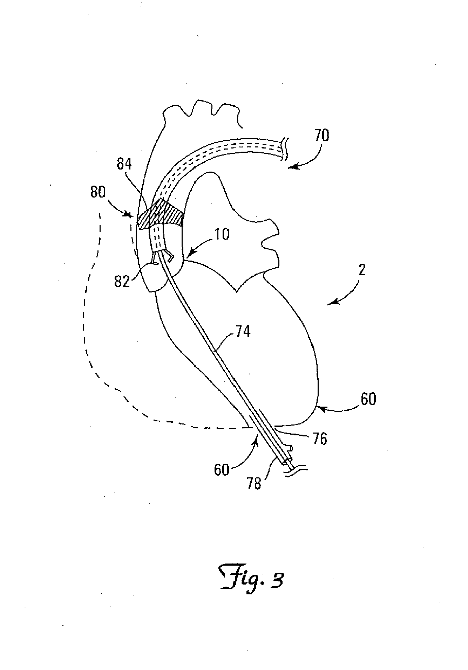

According to the embodiment of the present invention shown in FIG. 3, the valve access system 70 includes a leaflet removal (i.e., excision) module 80, which may include a leaflet cutting tool 82. The leaflet removal module 80 includes a rigid portion having an actuator exerting sufficient force to cut through a calcified leaflet. The actuator can be hydraulically or mechanically actuated to provide sufficient force to cut through stenotic native valve tissue. In various embodiments, the actuator is spring actuated. In yet another variant, the actuator is gas actuated. Exemplary leaflet removal or cutting tools are shown and described in U.S. Patent 5,304,189, U.S. Patent 5,370,685, and U.S. Publication 2006/0271081, each of which are incorporated herein by reference.

According to yet a further embodiment of the present invention, as is also shown in FIG. 3, the valve access system 70 also includes a leaflet capture device 84. The leaflet capture device 84 captures the removed portion or portions of the leaflet upon excision and traps any debris from the removal or decalcification of the leaflets. The capture device 84 entraps and contains the debris or excised portions. The capture device permits removal of the debris from the patient's heart 2.

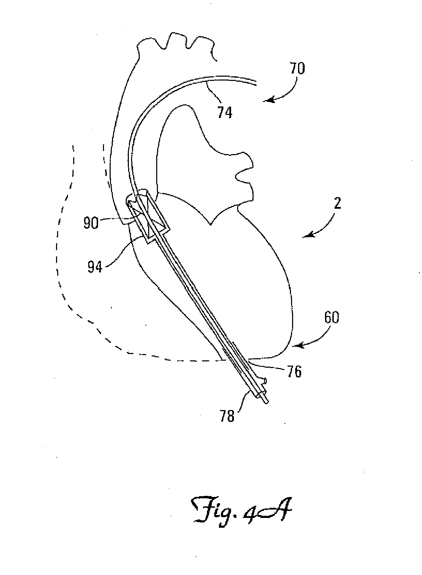

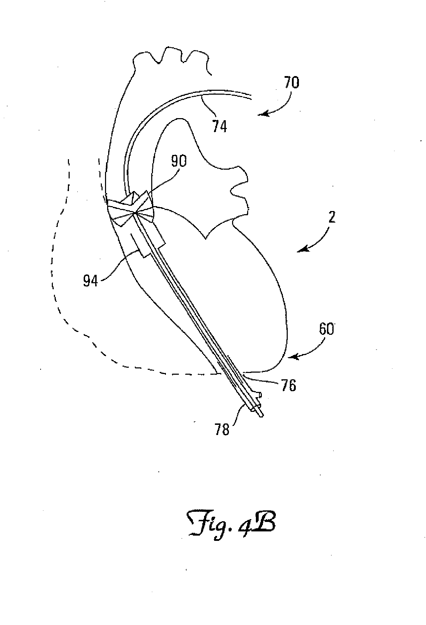

FIGS. 4A and 4B show another embodiment of the valve access system 70 of the present invention. As shown in FIG. 4A, the system 70 includes an expandable prosthetic heart valve 90 retained in a collapsed configuration within a delivery sheath 94. As described previously, the heart valve 90 can be crimped onto the delivery system 70 under water, such that the sheath is fluid filled to prevent embolism. Exemplary expandable prosthetic heart valves are shown and described in U.S. Publication 2006/0178740 and U.S. Publication 2005/0197695, both of which are incorporated herein by reference. An exemplary delivery system for an expandable prosthetic valve is shown and described in commonly-assigned, co-pending U.S. Application No. 11/612,980, entitled "Instrument and Method for In-Situ Deployment of Cardiac Valve Prosthesis", which is incorporated herein by reference. As shown in FIG. 4A, the prosthetic valve 90 has been delivered over the implantation device 74 to a target site within or near the aortic valve annulus 10 via an access port established at or near the apex 60. As shown, in FIG. 4B, the delivery sheath 94 can be retracted allowing the expandable prosthetic heart valve 90 to transition from a collapsed configuration to an expanded configuration, after which the implantation device 74 can be removed. According to other embodiments, the delivery sheath 94 could be introduced in a retrograde manner through the aorta or optionally through the aortic arch.

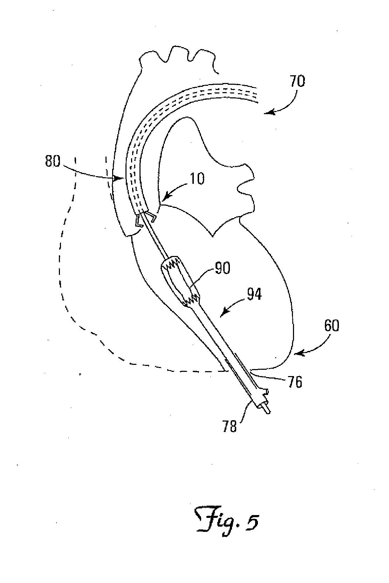

FIG. 5 shows an embodiment of the valve access system 70 of the present invention, which includes two interchangeable modules. As shown in FIG. 5, the valve access system 70 includes an implantation device 74 and a first module being delivered using an antegrade approach and a second module being delivered using a retrograde approach. According to one embodiment of the present invention, the modules have different functionalities. For example, in various embodiments, the first module is a leaflet cutter 80 and the second module is an expandable prosthetic valve 90 contained within a delivery sheath 94. After the leaflets have been excised from the valve annulus, the expandable prosthetic valve 90 can be quickly delivered and released to expand within the valve annulus. This configuration can help reduce the time that a patient's heart is beating, without a functioning valve in place, by allowing a physician to deliver both a valve removal device and a valve delivery device at or near the valve annulus 10, before actually removing the valve leaflets. The configuration is thus helpful for off-pump, beating heart prosthetic valve replacement.

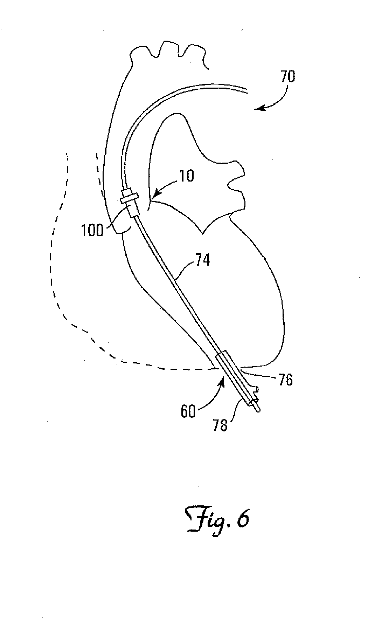

According to yet a further embodiment of the present invention, as shown in FIG. 6, the valve access system 70 includes a blood-pumping device 100. For example, the blood pumping device is a left ventricular assist device (LVAD) known to those of skill in the art. Exemplary LVADs are shown and described in U.S. Patent 7,144,364, which is incorporated herein by reference. The LVAD can be delivered using either an antegrade or a retrograde approach along the pathway established by the implantation device 74. The LVAD is then delivered to a target site within the left ventricle or the aorta.

In yet another variant of the invention, two distinct mechanically unconnected tracks are used upon which two mechanically unconnected modules are utilized. In this variant, a first module is configured to be delivered from a first opening in a patient's aortic tree and through the patient's aorta. One or more of the modules described herein may be used. A second module is configured to be delivered through an opening in a ventricle. Again, one or more modules described herein may be used. As with the other embodiments described herein, the fact that two simultaneous or nearly simultaneous access points are used on the patient allows for speed of native valve removal and implantation of a new prosthesis.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A device for implanting an expandable heart valve prosthesis at a valve annulus in a patient's heart, the device comprising:
an implantation device configured to extend from a first opening in the patient's body, through the patient's aorta, through a valve annulus, through an opening in a ventricle, and to exit through the patient's thoracic region; and
at least one module configured for delivery using the implantation device, and an optional second module.

2. The access system of claim 1, wherein at least one of the modules is selected from the group consisting of: an expandable heart valve prosthesis delivery module, a leaflet removal module, and a blood pumping module.

3. The access system of claim 2, wherein the leaflet excision module comprises a rigid portion having an actuator exerting sufficient force to cut through a calcified leaflet.

4. The access system of claim 1, wherein the implantation device is a guidewire.

5. The access system of claim 1, wherein the implantation device is a guide catheter.

6. The access system of claim 1, wherein the implantation device is structurally-continuous between the first opening and the second opening.

7. The access system of claim 1, wherein the modules can be delivered to the target location within the heart using either of an antegrade approach or a retrograde approach.

8. The access system of claim 1, wherein the implantation device is bendable and steerable.

9. The device of claim 8 in which the implantation device is reversibly lockable.

10. An access system for delivery of an expandable heart valve prosthesis to a patient's heart, the system comprising an implantation device configured to extend from a first opening in the patient's body, through the patient's aortal tree, through a valve annulus, through an opening in a ventricle, and to exit through the patient's thoracoabdominal region.

11. The system of claim 10 further comprising a port having a hemostasis valve for providing access into the ventricle.

12. The access system of claim 10 further comprising at least one interchangeable module capable of traveling along the implantation device to the valve annulus.

13. A method of implanting an expandable heart valve prosthesis comprising:
establishing a pathway extending from a first access port in a patient's body, through a valve annulus, and through a second access port in the patient's body;
delivering a first module from a first direction along the pathway, the first module configured to perform a first function; and
delivering a second module from a second direction along the pathway, the second module configured to perform a second function.

14. The method of claim 13 in which one of the first function and the second function is selected from the group consisting of: excising a portion of or all of a native leaflet, deploying an expandable heart valve prosthesis, and pumping blood.

15. The method of claim 13 further comprising providing a guidewire that extends to the outside of the patient through the first access port, the guidewire passing through an aorta and a ventricle of the patient, and the guidewire exiting the patient at the second access port.

16. A kit for implanting an expandable heart valve prosthesis, the kit comprising: a guidewire sized and dimensioned to enter a patient through a first opening or portal, extend through some or all of the patient's aorta and ventricle, and to exit the patient through a second opening or portal; and an expandable heart valve prosthesis delivery module.

17. The kit of claim 16 further comprising a leaflet removal module.

18. The kit of claim 17, wherein the leaflet removal module includes a leaflet capture device, the leaflet capture device adapted to capture and remove debris.

19. The kit of claim 18 further comprising a blood pumping module.

20. The kit of claim 16 further comprising a port for establishing access through an apex of the heart.

21. A device for implanting an expandable heart valve prosthesis in a patient's heart, the device comprising:
a first module configured to be delivered from a first opening in a patient's aortic tree and through the patient's aorta, and
a second module configured to be delivered through an opening in a ventricle, said first module free of mechanical connection to said second module.

22. The device of claim 21 in which said first or second module is selected from the group consisting of: a ballooning module, a cutting module, an expandable valve prosthesis module, and a debris catching module.
